# EUROPEAN PATENT APPLICATION

(11) **EP 4 544 990 A1**
(43) Date of publication of application: **30.04.2025**
(21) Application number: 24206225.5
(22) Date of filing: 11.10.2024
(51) Int. Cl.: A61B 5/00, G16H 40/63, H04B 10/114

(54) **MEASUREMENT SYSTEM**

(30) Priority: 13.10.2023 JP 2023177879
(71) Applicant: ARKRAY, Inc., Kyoto-shi, Kyoto 601-8045 (JP)
(72) Inventor: ARIMA, Hiroki, Kyoto-shi, 602-0008 (JP); MUROTSUKI, Kei, Kyoto-shi, 602-0008 (JP)
(74) Representative: Dehns

(57) **Abstract**

A measurement system (1) includes: a measurement device (10) that includes a first communication unit (21) and a second communication unit (22) and acquires a measurement result of biological information of a user, the first communication unit performing communication by a first communication method and the second communication unit performing communication by a second communication method different from the first communication method; a terminal device (30) that includes a terminal communication unit (37) that performs communication with the first communication unit of the measurement device, and a terminal notification unit (36) that notifies of the measurement result acquired by the terminal communication unit; and a notification device (50) that includes a notification communication unit (57) that performs communication with the second communication unit of the measurement device by the second communication method, and a notification unit that notifies of the measurement result acquired by the notification communication unit.

## Description

### BACKGROUND

### Technical Field

The present invention relates to a measurement system.

### Related Art

For example, a measurement device that measures a blood glucose level in blood and does not include a display that displays a measurement result in order to improve portability by downsizing has been known (Japanese Patent No. 5535251). The measurement device described in Japanese Patent No. 5535251 performs wireless communication such as Bluetooth (registered trademark) with a terminal device such as a smartphone to display the measurement result on the terminal device. The measurement device can also perform wired communication, and Japanese Patent No. 5535251 describes that the measurement device and a computer are electrically connected by a cable, and the measurement result is transmitted to the computer and stored. Meanwhile, a notification device that is connected to a measurement device in a wired manner and provides notification of a measured value by reading the measured value has been proposed (see Japanese Patent No. 4707296).

It is desirable for the above-described measurement device that does not include the display that displays measurement result to adaptively notify of the measurement result according to a use situation.

### SUMMARY

The present invention has been made in view of the circumstances, at least preferred embodiments of the invention adaptively notify of a measurement result according to a use situation of a measurement device.

A measurement system according to the present invention includes:
a measurement device that includes a first communication unit and a second communication unit and that acquires a measurement result of biological information of a user, the first communication unit performing communication by a first communication method and the second communication unit performing communication by a second communication method different from the first communication method;
a terminal device that includes a terminal communication unit that performs communication with the first communication unit of the measurement device, and a terminal notification unit that provides notification of the measurement result acquired by the terminal communication unit; and
a notification device that includes a notification communication unit that performs communication with the second communication unit of the measurement device by the second communication method, and a notification unit that provides notification of the measurement result acquired by the notification communication unit.

Accordingly, it is possible to adaptively notify of a measurement result according to a use situation of the measurement device.

### BRIEF DESCRIPTION OF THE DRAWINGS

Fig. 1 is a schematic view illustrating an example of a measurement system according to a first embodiment;
Fig. 2 is a block diagram illustrating an example of a hardware configuration of the measurement device;
Fig. 3 is a block diagram illustrating an example of a functional configuration of the measurement device;
Fig. 4 is a block diagram illustrating an example of a hardware configuration of a terminal device;
Fig. 5 is a block diagram illustrating an example of a functional configuration of the terminal device;
Fig. 6 is a block diagram illustrating an example of a hardware configuration of a notification device;
Fig. 7 is a block diagram illustrating an example of a functional configuration of the notification device;
Fig. 8 is a schematic view illustrating a state in which the measurement device and the notification device are connected;
Fig. 9 is a sequence diagram illustrating an example of processing executed in a case in which the measurement device and the notification device are connected in a measurement system according to the embodiment;
Fig. 10 is a sequence diagram illustrating an example of processing executed in the case of displaying a measurement result on the terminal device in the measurement system according to the embodiment;
Fig. 11 is a schematic view illustrating an example of a measurement system according to a second embodiment;
Fig. 12 is a block diagram illustrating an example of a hardware configuration of a communication device;
Fig. 13 is a block diagram illustrating an example of a functional configuration of the communication device; and
Fig. 14 is a sequence diagram illustrating an example of processing executed in the measurement system according to the second embodiment in a case in which the measurement device and the communication device are connected.

### DETAILED DESCRIPTION

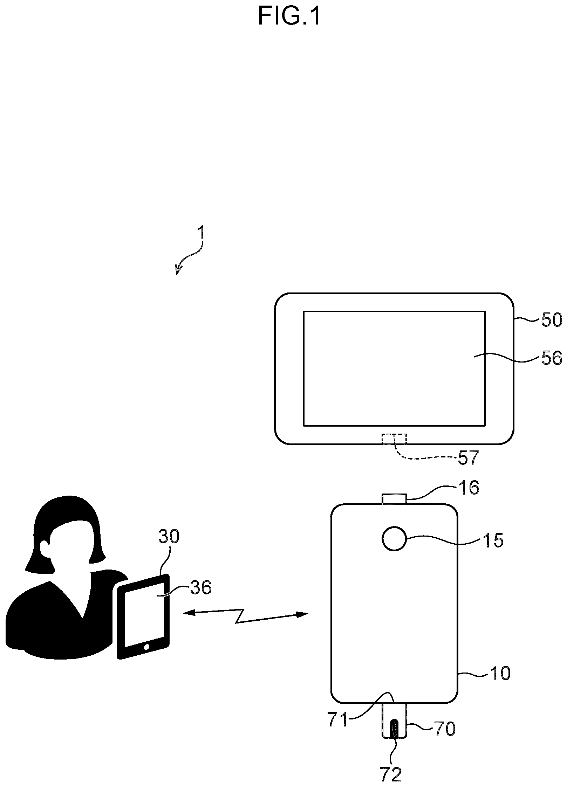

Hereinafter, embodiments of the invention will be described in detail with reference to the drawings. Fig. 1 is a schematic diagram illustrating an example of a configuration of a measurement system 1 according to a first embodiment.

As illustrated in Fig. 1, the measurement system 1 includes a measurement device 10, a terminal device 30, and a notification device 50. The measurement device 10 and the notification device 50 can perform wired communication by being electrically connected by a connector 16 of the measurement device 10 and a connector 57 of the notification device 50. For example, a universal serial bus (USB) connector can be used as the connectors 16 and 57. The measurement device 10 and the terminal device 30 can wirelessly communicate with each other via, for example, Bluetooth (registered trademark) Low Energy (BLE) communication. The BLE communication is an example of a first communication method, and electrical connection is an example of a second communication method.

The measurement device 10 measures biological information of a user. Here, examples of the biological information include information indicating a blood glucose level, a blood oxygen concentration, a hemoglobin level, a body temperature, a heart rate, an electrocardiogram waveform, a blood pressure, and the like, and the biological information may be any biological information that can be measured from a human body. Hereinafter, a case in which the measurement device 10 is a blood glucose measurement device that measures the blood glucose level of the user will be specifically described as an example. For example, the measurement device 10 includes a sensor strip 70 for measuring the blood glucose level and an insertion port 71 into which the sensor strip 70 is inserted, and the sensor strip 70 includes an application portion 72 where the blood of the user is applied. Here, the measurement device 10 includes a terminal (not illustrated) that is energized when the sensor strip 70 is inserted into the insertion port 71.

In the measurement device 10, a measurement region (not illustrated) communicating with the application portion 72 of the sensor strip 70 through a flow path is energized when the blood is applied to the application portion 72 of the sensor strip 70 inserted into the insertion port 71, and the blood glucose level in the blood is electrically measured based on a response current value. The measurement device 10 continues to apply a voltage to the measurement region after the sensor strip 70 is inserted into the insertion port 71, and when the blood reaches the measurement region, a liquid junction is formed and the energization is made. "Applying" means attaching (adsorbing) the blood of the user to the application portion 72.

The measurement device 10 does not include a display for displaying a measurement result including the measured blood glucose level or the like, and includes a light emitting diode (LED) 15 for notifying of a simple measurement result.

The measurement device 10 transmits the measurement result to the terminal device 30 through the BLE communication. The terminal device 30 includes a display 36, and displays the received measurement result on the display 36. The terminal device 30 may include means for notifying of the measurement result by voice, such as a speaker, instead of or in addition to the display 36.

When connected to the notification device 50, the measurement device 10 transmits the measurement result to the notification device 50. The notification device 50 includes a display 56, and notifies of the measurement result by displaying the received measurement result on the display 56. The notification device 50 may include means for notifying of the measurement result by voice, such as a speaker, instead of or in addition to the display 56.

The terminal device 30 is a terminal such as a mobile terminal such as a smartphone, a tablet, or a personal computer that can communicate with an external device. The terminal device 30 performs wireless communication with the measurement device 10 through BLE communication. The terminal device 30 transmits, for example, a connection request including a passcode necessary for connection to the measurement device 10 to the measurement device 10. The measurement device 10 transmits a connection response to the terminal device 30 to establish communication with the terminal device 30 by authenticating the passcode. When the first communication is established, the terminal device 30 shares an encryption key used when performing communication with the measurement device 10 (performs pairing) with the measurement device 10, and stores (bonds) the encryption key.

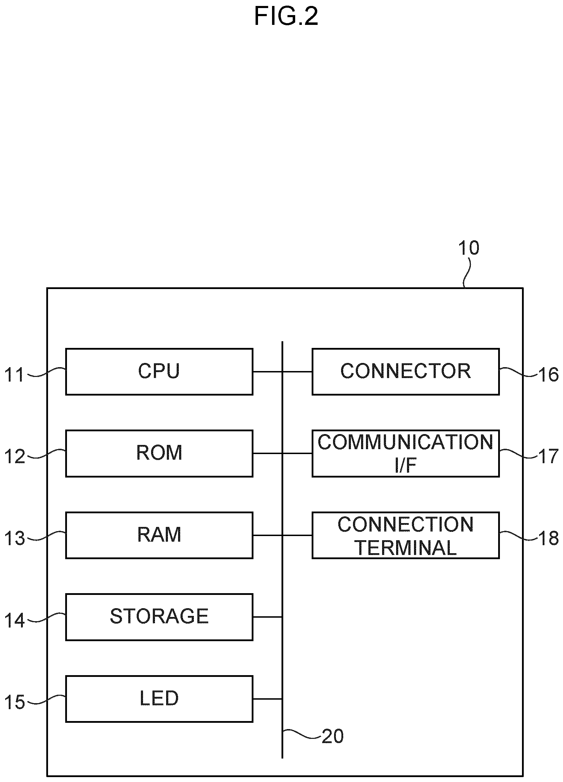

Next, a hardware configuration of the measurement device 10 will be described. Fig. 2 is a block diagram illustrating an example of a hardware configuration of the measurement device. As illustrated in Fig. 2, the measurement device 10 includes a central processing unit (CPU) 11, a read only memory (ROM) 12, a random access memory (RAM) 13, a storage 14, the LED 15, a connector 16, a communication interface (communication I/F) 17, and a connection terminal 18. The CPU 11, the ROM 12, the RAM 13, the storage 14, the LED 15, the connector 16, the communication I/F 17, and the connection terminal 18 are mutually connected by a bus 20.

The CPU 11 integrally controls the entire measurement device 10. The ROM 12 stores various programs, data, and the like. The RAM 13 is a memory used as a work area when various programs are executed. The CPU 11 loads a program stored in the ROM 12 in the RAM 13 and executes the program to execute various types of processing such as measurement of the blood glucose level and communication.

The storage 14 is, for example, a hard disk drive (HDD), a solid state drive (SSD), or a flash memory. The storage 14 may store various programs and the like.

The LED 15 simply notifies of the measurement result of the blood glucose level, and for example, lights up in red in a case in which a measured value is higher than a reference range, lights up in green in a case in which the measured value is within the reference range, and lights up in blue in a case in which the measured value is lower than the reference range. Therefore, it is possible to notify the user of a simple measurement result of the blood glucose level.

The connector 16 is electrically connected to the connector 57 of the notification device 50 to transmit and receive data. The communication I/F 17 transmits and receives data by using wireless communication conforming to a BLE standard. The communication I/F 17 is an example of a first communication unit, and the connector 16 is an example of a second communication unit.

The connection terminal 18 is electrically connected to the sensor strip 70 in which the blood is applied to the application portion 72. A measurement control unit 23 described below acquires the blood glucose level based on the response current value generated here.

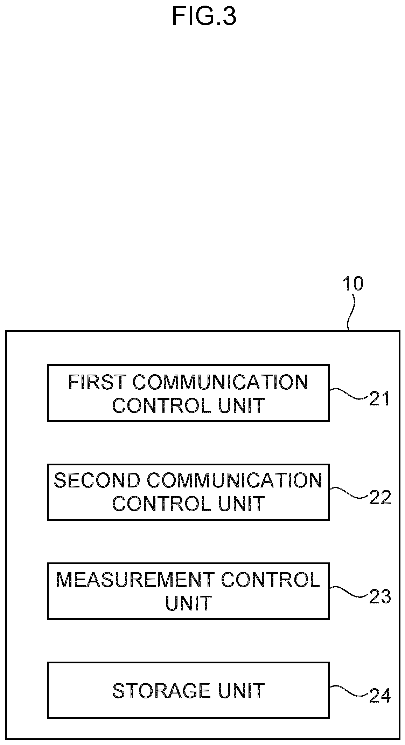

Next, a functional configuration of the measurement device 10 will be described. Fig. 3 is a block diagram illustrating an example of the functional configuration of the measurement device 10. As illustrated in Fig. 3, the measurement device 10 includes a first communication control unit 21, a second communication control unit 22, a measurement control unit 23, and a storage unit 24. When the CPU 11 executes various programs stored in the ROM 12, the CPU 11 functions as the first communication control unit 21, the second communication control unit 22, the measurement control unit 23, and the storage unit 24.

The first communication control unit 21 performs communication with the terminal device 30 via the communication I/F 17. For example, when establishing the first communication with the terminal device 30, the first communication control unit 21 performs two-way communication with the terminal device 30 and authenticates the passcode included in the connection request transmitted from the terminal device 30. After the authentication, the connection response is transmitted to the terminal device 30. As a result, the first communication control unit 21 establishes the first communication with the terminal device 30.

After the first communication is established, the first communication control unit 21 shares an encryption key used when performing communication with the measurement device 10 (performs pairing) with the measurement device 10, and stores (bonds) the encryption key.

In the second and subsequent connections, the first communication control unit 21 determines whether or not to establish communication with the terminal device 30 by using connection information transmitted from the terminal device 30, and transmits the connection response to the terminal device 30 to establish communication with the terminal device 30 in the case of establishing communication.

In the embodiment, the first communication control unit 21 monitors whether or not the measurement device 10 is in communication with the notification device 50. The phrase "the measurement device 10 is in communication with the notification device 50" means that the second communication control unit 22 described below recognizes connection between the measurement device 10 and the notification device 50. A state in which the connection is recognized includes, for example, not only a state in which the measurement device 10 and the notification device 50 are actually in communication with each other by connection between the connectors 16 and 57 as in the embodiment, but also a state in which the measurement device 10 and the notification device 50 are only physically connected by the connectors 16 and 57 and communication is not performed between the measurement device 10 and the notification device 50. In a case in which the measurement device 10 is in communication with the notification device 50, the first communication control unit 21 stops communication with the terminal device 30. The phrase "stopping communication" means that communication is disconnected in a case in which the measurement device 10 is in communication with the terminal device 30, and means that communication is prohibited even when there is a connection request from the terminal device 30 in a case in which the measurement device 10 is not in communication with the terminal device 30. Therefore, in the embodiment, the first communication control unit 21 performs communication with the terminal device 30 only in a case in which the measurement device 10 and the notification device 50 are not connected via the connectors 16 and 57.

The second communication control unit 22 performs wired communication with the notification device 50 by being electrically connected to the notification device 50 via the connector 16.

When the blood is applied to the sensor strip 70 inserted into the insertion port 71 and the above-described measurement region is energized by the connection terminal 18, the measurement control unit 23 measures the response current value generated here. Then, the measurement control unit 23 converts the measured response current value into a voltage, and further converts the voltage to acquire the measurement data indicating the measurement result of the blood glucose level. The measurement control unit 23 stores the measurement data in the storage 14. In addition, the measurement control unit 23 turns on the LED 15 in a color corresponding to a value of the measurement result.

The storage unit 24 performs bonding for storing the encryption key of the terminal device 30 paired by the first communication control unit 21.

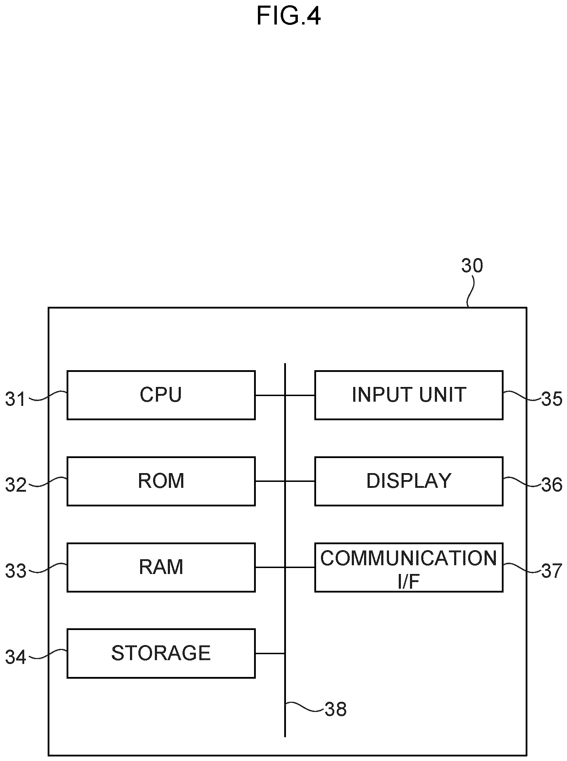

Next, a hardware configuration of the terminal device 30 will be described. Fig. 4 is a block diagram illustrating an example of the hardware configuration of the terminal device. As illustrated in Fig. 4, the terminal device 30 includes a CPU 31, a ROM 32, a RAM 33, a storage 34, an input unit 35, the display 36, and a communication I/F 37. The CPU 31, the ROM 32, the RAM 33, the storage 34, the input unit 35, the display 36, and the communication I/F 37 are mutually connected by a bus 38.

The CPU 31 integrally controls the entire terminal device 30. The ROM 32 stores various programs, data, and the like. The RAM 33 is a memory used as a work area when various programs are executed. The CPU 31 loads a program stored in the ROM 32 in the RAM 33 and executes the program to execute various types of processing such as communication with the measurement device 10.

The storage 34 is, for example, an HDD, an SSD, or a flash memory. The storage 34 may store various programs and the like.

The input unit 35 is a keyboard and a pointing device that receive input of characters, selection of an image, and the like. The display 36 displays characters and images. The communication I/F 37 transmits and receives data by using wireless communication conforming to the BLE standard. The display 36 is an example of a terminal notification unit, and the communication I/F 37 is an example of a terminal communication unit.

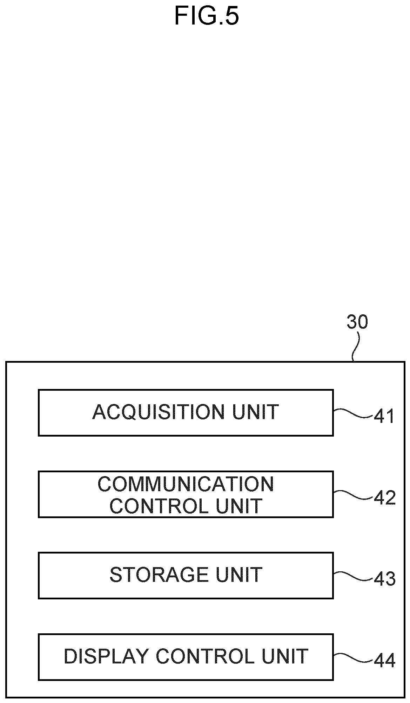

Next, a functional configuration of the terminal device 30 will be described. Fig. 5 is a block diagram illustrating an example of the functional configuration of the terminal device 30. As illustrated in Fig. 5, the terminal device 30 includes an acquisition unit 41, a communication control unit 42, a storage unit 43, and a display control unit 44. When the CPU 31 executes various programs stored in the ROM 32, the CPU 31 functions as the acquisition unit 41, the communication control unit 42, the storage unit 43, and the display control unit 44. A mode in which each function is implemented as a function included in the terminal device 30 by executing a program for establishing communication stored in advance as an operation system will be described. However, the invention is not limited thereto. For example, the program for establishing communication may be stored in the terminal device 30 in advance as an application that can be acquired by being downloaded from an external device, and each function may be implemented by executing the application.

The acquisition unit 41 acquires the measurement data indicating the measurement result transmitted from the measurement device 10.

The communication control unit 42 performs communication with the measurement device 10 via the communication I/F 37, and transmits and receives data to and from the measurement device 10. In the case of establishing the first communication, the communication control unit 42 performs bidirectional communication with the communication I/F 37 of the measurement device 10 via the communication I/F 17, and transmits the connection request including the passcode for connection to the measurement device 10. When the measurement device 10 authenticates the passcode, and the connection response transmitted from the measurement device 10 is received, the communication control unit 42 establishes communication with the measurement device 10.

After the establishment of the first communication, the communication control unit 42 performs pairing of the encryption key with the measurement device 10 and bonds the encryption key.

In the second and subsequent connections, the communication control unit 42 transmits the connection request to the measurement device 10 and establishes communication with the measurement device 10 based on the connection response transmitted from the measurement device 10.

The storage unit 43 stores the encryption key acquired from the measurement device 10.

The display control unit 44 displays the measurement result acquired from the measurement device 10 on the display 36.

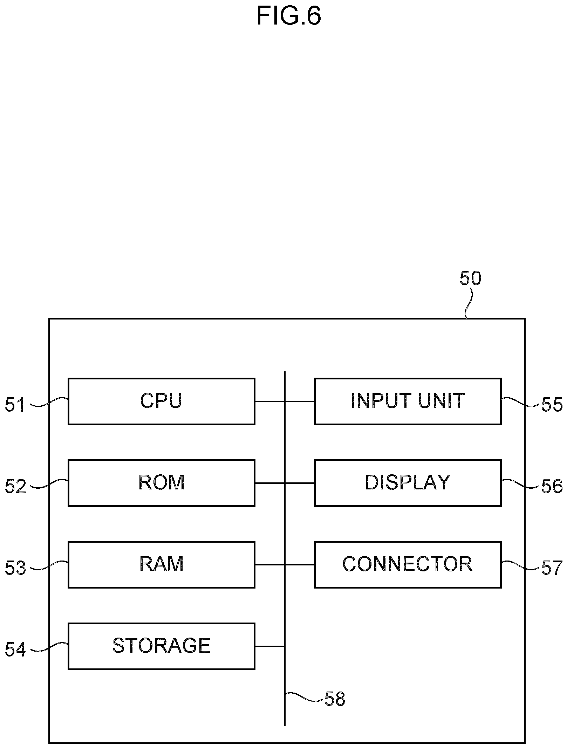

Next, a hardware configuration of the notification device 50 will be described. Fig. 6 is a block diagram illustrating an example of the hardware configuration of the notification device. As illustrated in Fig. 6, the notification device 50 includes a CPU 51, a ROM 52, a RAM 53, a storage 54, an input unit 55, the display 56, and a connector 57. The CPU 51, the ROM 52, the RAM 53, the storage 54, the input unit 55, the display 56, and the connector 57 are mutually connected by a bus 58.

The CPU 51 integrally controls the entire notification device 50. The ROM 52 stores various programs, data, and the like. The RAM 53 is a memory used as a work area when various programs are executed. The CPU 51 loads various programs stored in the ROM 52 in the RAM 53 and executes the programs to perform communication with the measurement device 10 and execute processing such as displaying the measurement result on the display 56.

The storage 54 is, for example, an HDD, an SSD, or a flash memory. The storage 54 may store various programs and the like.

The input unit 55 is a button that receives input, selection, and the like. The connector 57 is electrically connected to the connector 16 of the measurement device 10 to transmit and receive data. The display 56 is an example of a notification unit, and the connector 57 is an example of a notification communication unit.

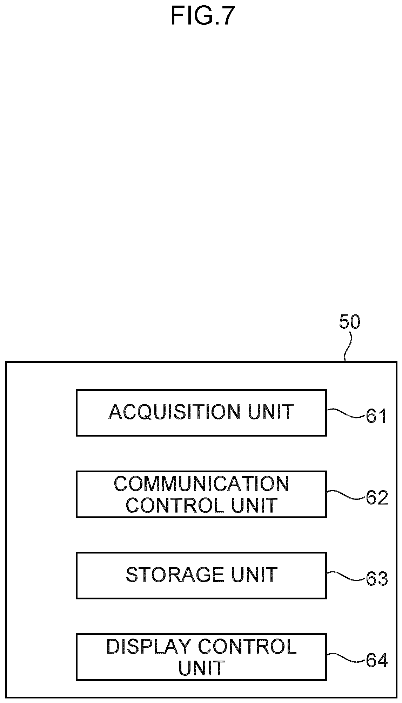

Next, a functional configuration of the notification device 50 will be described. Fig. 7 is a block diagram illustrating an example of the functional configuration of the notification device 50. As illustrated in Fig. 7, the notification device 50 includes an acquisition unit 61, a communication control unit 62, a storage unit 63, and a display control unit 64. When the CPU 51 executes various programs stored in the ROM 52, the CPU 51 functions as the acquisition unit 61, the communication control unit 62, the storage unit 63, and the display control unit 64.

The acquisition unit 61 acquires the measurement data indicating the measurement result transmitted from the measurement device 10.

The communication control unit 62 performs wired communication with the measurement device 10 by being electrically connected to the measurement device 10 via the connector 57.

The storage unit 63 stores the measurement result transmitted from the measurement device 10.

The display control unit 64 makes a notification by displaying the measurement result transmitted from the measurement device 10 on the display 56.

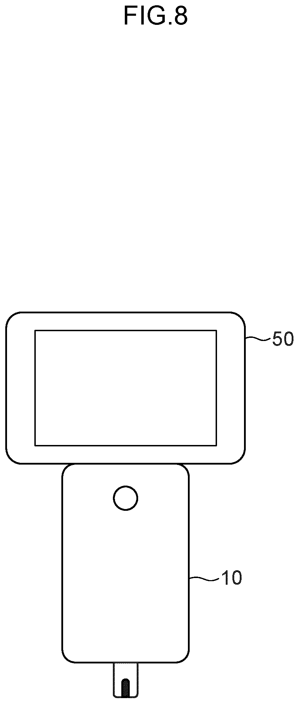

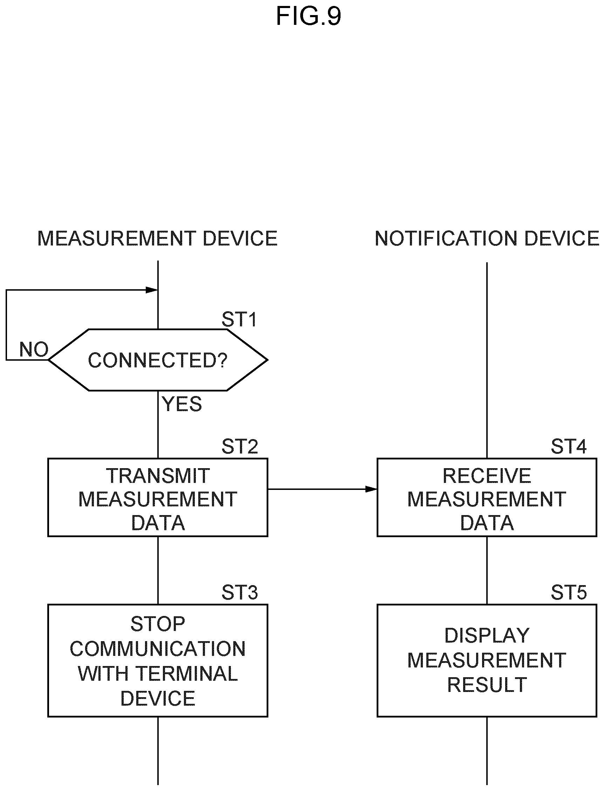

Next, processing executed in the first embodiment will be described. First, as illustrated in Fig. 8, processing in a case in which the measurement device 10 and the notification device 50 are connected will be described. Fig. 9 is a sequence diagram illustrating an example of processing executed in the measurement system according to the first embodiment in a case in which the measurement device 10 and the notification device 50 are connected. In the following description, it is assumed that the blood glucose level is measured in the measurement device 10 and the measurement data indicating the measurement result is acquired.

The measurement device 10 monitors whether or not the notification device 50 is connected (step ST1), and in a case in which a result of step ST1 is affirmative, the measurement device 10 transmits the measurement data indicating the measurement result to the notification device 50 (step ST2). In addition, the measurement device 10 stops communication with the terminal device 30 (step ST3). The notification device 50 receives the measurement data transmitted from the measurement device 10 (step ST4) and displays the measurement data on the display 56 (step ST5).

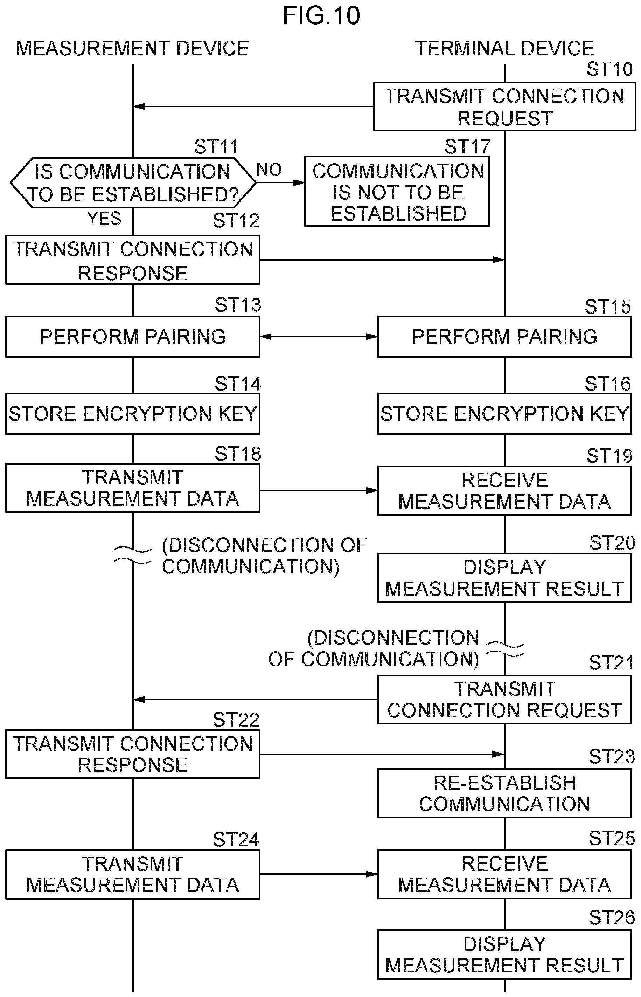

Next, processing in the case of displaying the measurement result on the terminal device 30 will be described. Fig. 10 is a sequence diagram illustrating an example of processing executed in the case of displaying the measurement result on the terminal device 30 in the measurement system according to the present embodiment.

First, the terminal device 30 transmits the connection request to the measurement device 10 (step ST10). The measurement device 10 determines whether or not to establish communication by authenticating the connection request (step ST11). In the case of establishing communication (step ST11: YES), the measurement device 10 transmits the connection response to the terminal device 30 (step ST12). Then, the measurement device 10 establishes communication, performs pairing (step ST13), and stores the encryption key (step ST14). The terminal device 30 similarly performs pairing (step ST15) and stores the encryption key (step ST16). In the case of not establishing communication (step ST11: NO), the measurement device 10 does not transmit the connection response to the terminal device 30 and does not establish communication with the terminal device 30 (step ST17).

Next, the measurement device 10 transmits the measurement data indicating the measurement result to the terminal device 30 (step ST18). The terminal device 30 receives the measurement data (step ST19) and displays the measurement result on the display 36 (step ST20).

In a case in which communication with the terminal device 30 is performed again to transmit the measurement result to the terminal device 30 after the communication is disconnected, the terminal device 30 transmits the connection request to the measurement device 10 in order to establish communication with the measurement device 10 (step ST21). The measurement device 10 transmits the connection response for the received connection request (step ST22). In a case in which the connection response is received, the terminal device 30 re-establishes communication with the measurement device 10 (step ST23). Then, the measurement device 10 transmits the measurement data indicating the measurement result to the terminal device 30 (step ST24). The terminal device 30 receives the measurement data (step ST25) and displays the measurement result on the display 36 (step ST26).

As described above, in the embodiment, the measurement device 10 and the notification device 50 can be connected according to a use situation of the measurement device 10 and the measurement result can be displayed on the notification device 50, or the measurement result can be displayed on the terminal device 30 by performing communication with the terminal device 30. Therefore, it is possible to adaptively notify of the measurement result according to the use situation of the measurement device 10. For example, when the user is at home, the measurement result can be immediately displayed only by connecting the notification device 50 to the measurement device 10. In addition, in a situation where the notification device 50 is not carried, such as a situation in which the user is out, the measurement result can be displayed in a manner in which the measurement data is transmitted from the measurement device 10 to the terminal device 30.

In a case in which the measurement device 10 and the notification device 50 are connected, communication between the measurement device 10 and the terminal device 30 is stopped. Therefore, it is possible to prevent the measurement device 10 from erroneously performing communication with the terminal device 30 in a situation where wireless communication is prohibited, for example, in the case of boarding an airplane. Even in a case in which the measurement device 10 and the terminal device 30 cannot perform communication with each other, the measurement result can be displayed on the notification device 50 by connecting the measurement device 10 and the notification device 50.

In the first embodiment, the measurement device 10 and the notification device 50 are electrically connected by the connectors 16 and 57 to perform wired communication between the measurement device 10 and the notification device 50, but the invention is not limited thereto. Any communication method can be used as long as the communication method is different from the communication method between the measurement device 10 and the terminal device 30. For example, an optical communication method such as an infrared communication method, non-contact Ethernet (registered trademark) communication, or the like can be used.

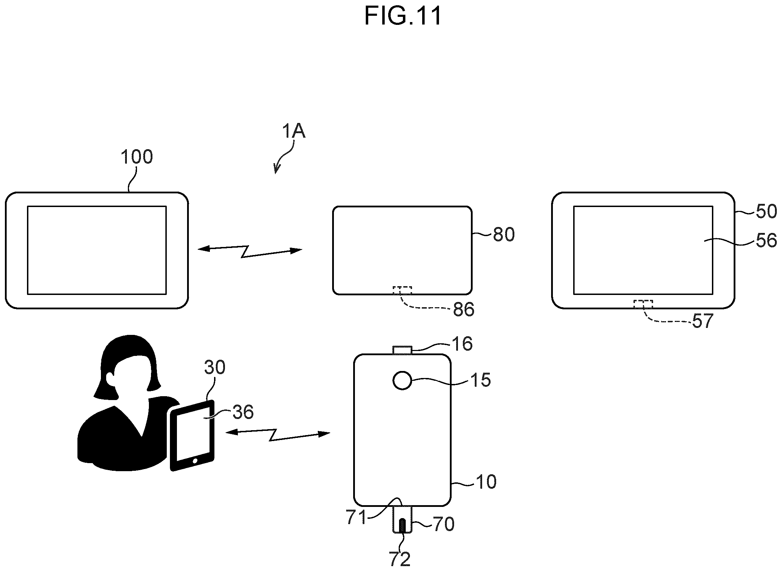

Next, a second embodiment of a measurement system will be described. Fig. 11 is a schematic diagram illustrating an example of a configuration of the measurement system according to the second embodiment. In the second embodiment, the same reference numerals are given to the same components as those of the first embodiment, and a detailed description thereof will be omitted. As illustrated in Fig. 11, a measurement system 1A according to the second embodiment includes a communication device 80 in addition to a measurement device 10, a terminal device 30, and a notification device 50.

The communication device 80 includes a connector 86 that is the same as a connector 57 of the notification device 50. The measurement device 10 and the communication device 80 can perform wired communication by being electrically connected by a connector 16 of the measurement device 10 and the connector 86 of the communication device 80.

In the second embodiment, the communication device 80 performs communication with, for example, a display device 100 in a hospital, thereby transmitting measurement data indicating a measurement result acquired by the measurement device 10 to, for example, the display device 100 in the hospital. Communication between the communication device 80 and the display device 100 is performed by, for example, an in-hospital network installed in the hospital. A communication method in the in-hospital network is a communication method different from BLE communication between the measurement device 10 and the terminal device 30, and for example, low power wide area (LPWA), near field communication (NFC), or Wi-Fi is used. The communication method in the in-hospital network is an example of a third communication method. The in-hospital network is, for example, a special network installed in the hospital in order to increase a security level, and connection to the in-hospital network cannot be made by a communication method using a communication I/F 17 included in the measurement device 10.

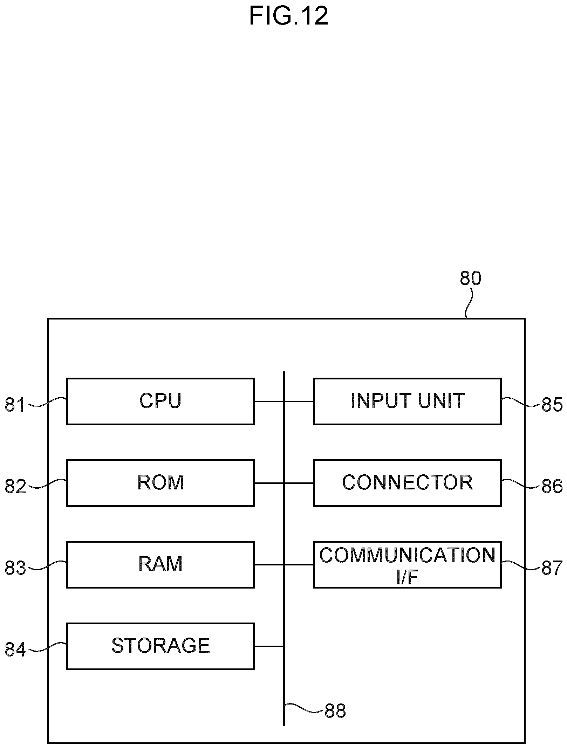

Next, a hardware configuration of the communication device 80 will be described. Fig. 12 is a block diagram illustrating an example of the hardware configuration of the communication device. As illustrated in Fig. 12, the communication device 80 includes a CPU 81, a ROM 82, a RAM 83, a storage 84, an input unit 85, the connector 86, and a communication I/F 87. The CPU 81, the ROM 82, the RAM 83, the storage 84, the input unit 85, the connector 86, and the communication I/F 87 are mutually connected by a bus 88.

The CPU 81 integrally controls the entire communication device 80. The ROM 82 stores various programs, data, and the like. The RAM 83 is a memory used as a work area when various programs are executed. The CPU 81 loads various programs stored in the ROM 82 in the RAM 83 and executes the programs to execute processing such as communication with the measurement device 10 and the display device 100 in the hospital.

The storage 84 is, for example, an HDD, an SSD, or a flash memory. The storage 84 may store various programs and the like.

The input unit 85 is a button that receives input, selection, and the like. The connector 86 is electrically connected to the connector 16 of the measurement device 10 to transmit and receive data. The communication I/F 87 is connected to the in-hospital network to transmit and receive data to and from the display device 100 by using wireless communication.

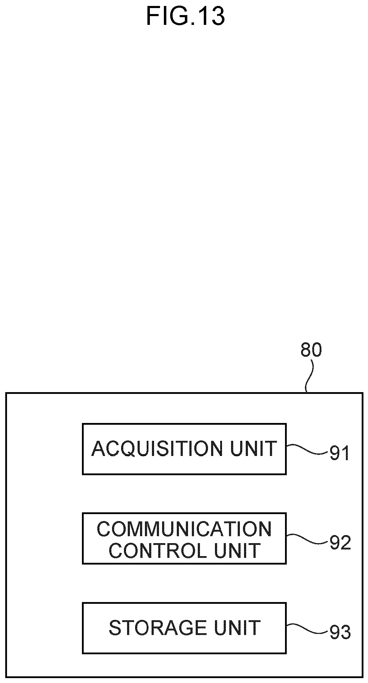

Next, a functional configuration of the communication device 80 will be described with reference to Fig. 13. Fig. 13 is a block diagram illustrating an example of the functional configuration of the communication device 80. As illustrated in Fig. 13, the communication device 80 includes an acquisition unit 91, a communication control unit 92, and a storage unit 93. When the CPU 81 executes various programs stored in the ROM 82, the CPU 81 functions as the acquisition unit 91, the communication control unit 92, and the storage unit 93.

The acquisition unit 91 acquires the measurement data indicating the measurement result transmitted from the measurement device 10.

The communication control unit 92 performs wired communication with the measurement device 10 by being electrically connected to the measurement device 10 via the connector 86. In addition, wireless communication is performed with the display device 100 via the communication I/F 87.

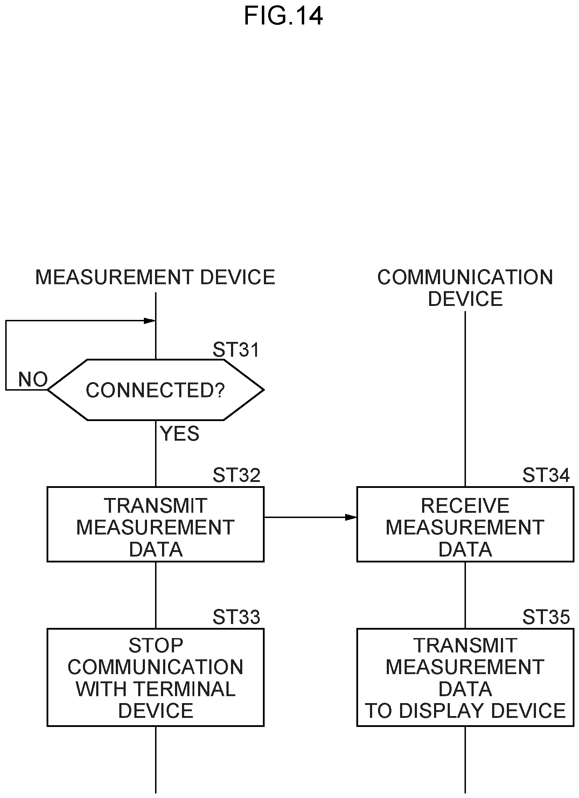

Next, processing executed in the second embodiment will be described. Fig. 14 is a sequence diagram illustrating an example of processing executed in the measurement system according to the second embodiment in a case in which the measurement device 10 and the communication device 80 are connected. It is assumed that the blood glucose level is measured in the measurement device 10 and the measurement data indicating the measurement result is acquired.

The measurement device 10 monitors whether or not the communication device 80 is connected (step ST31), and in a case in which a result of step ST31 is affirmative, the measurement data indicating the measurement result is transmitted to the communication device 80 (step ST32). In addition, the measurement device 10 stops communication with the terminal device 30 (step ST33). The communication device 80 receives the measurement data transmitted from the measurement device 10 (step ST34) and transmits the measurement data to the display device 100 (step ST35). Although not illustrated in Fig. 14, the display device 100 receives the measurement data and displays the measurement result.

As described above, in addition to the configuration according to the first embodiment, in the second embodiment, the measurement device 10 is connected to the communication device 80, and the measurement result is displayed on the display device 100 in the hospital by using the communication device 80 and, for example, the in-hospital network. For this reason, even when the user is in a situation in which only the communication method unavailable for the measurement device 10 is available, in a case in which the communication device 80 capable of performing communication by the communication method unavailable for the measurement device 10 is prepared, the measurement result of the measurement device 10 can be transmitted to the display device 100 and displayed. Therefore, adaptability to a use situation of the measurement device 10 can be further improved.

In the second embodiment, the measurement device 10 and the communication device 80 are electrically connected by the connectors 16 and 86 to perform wired communication between the measurement device 10 and the communication device 80, but the invention is not limited thereto. Any communication method can be used as long as the communication method is different from the communication method between the measurement device 10 and the terminal device 30. For example, an optical communication method such as an infrared communication method, non-contact Ethernet communication, or the like can be used.

In each embodiment, the measurement result is displayed on the display 56 in the notification device 50, but the invention is not limited thereto. The notification device 50 may include means for outputting a sound, such as a speaker, and the measurement result may be notified by the sound instead of or in addition to the display of the measurement result.

In each embodiment, the measurement device 10 simply displays the measurement result by using the LED 15, but the invention is not limited thereto. The invention can also be applied to the measurement device 10 that does not include the LED 15.

In each embodiment, the measurement device 10 is a blood glucose measurement device, but the invention is not limited thereto. The measurement device 10 may be a blood pressure measurement device or a body temperature measurement device, or may be any device as long as data can be transmitted and received by performing communication with the notification device 50 and data can be transmitted and received using the BLE communication with the external terminal device 30.

In each embodiment, the processor refers to a processor in a broad sense, and includes a general-purpose processor (for example, a CPU) and a dedicated processor (for example, a graphics processing unit (GPU), an application specific integrated circuit (ASIC), a field programmable gate array (FPGA), or a programmable logic device).

In addition, the operation of the processor in each embodiment may be performed not only by one processor but also by a plurality of processors existing at physically separated positions in cooperation. In addition, the order of the respective operations of the processor is not limited to the order described in each embodiment, and may be changed, if appropriate.

In addition, the configurations of the measurement device, the terminal device, and the communication device described in the embodiments are merely examples, and may be changed according to the situation without departing from the scope of the present invention, as defined by the claims.

In addition, the flow of processing of the program described in the embodiments is also an example, and unnecessary steps may be deleted, new steps may be added, or the processing order may be changed without departing from the scope of the present invention.

Furthermore, in the embodiments, a case in which the processing according to the embodiment is implemented by a software configuration using a computer by executing a program has been described, but the invention is not limited thereto. The embodiments may be implemented by, for example, a hardware configuration or a combination of a hardware configuration and a software configuration.

## Claims

1. A measurement system (1; 1A), comprising:
a measurement device (10) that includes a first communication unit (21) and a second communication unit (22) and that is configured to acquire a measurement result of biological information of a user, the first communication unit being configured to perform communication by a first communication method and the second communication unit being configured to perform communication by a second communication method different from the first communication method;
a terminal device (30) that includes a terminal communication unit (37) that is configured to perform communication with the first communication unit of the measurement device, and a terminal notification unit (36) that is configured to provide notification of the measurement result acquired by the terminal communication unit; and
a notification device (50) that includes a notification communication unit (57) that is configured to perform communication with the second communication unit of the measurement device by the second communication method, and a notification unit (56) that is configured to provide notification of the measurement result acquired by the notification communication unit.

2. The measurement system according to claim 1, wherein the first communication unit (21) is configured to stop communication with the terminal device (30) in a case in which the measurement device (10) is in communication with the notification device (50) by the second communication unit (22).

3. The measurement system according to claim 1 or 2, wherein the second communication method is a communication method based on electrical connection between the measurement device (10) and the notification device (50).

4. The measurement system according to claim 1 or 2, wherein the second communication method is an optical communication method between the measurement device (10) and the notification device (50).

5. The measurement system according to claim 4, wherein the optical communication method is an infrared communication method.

6. The measurement system according to claim 1 or 2, wherein the second communication method is a communication method based on non-contact Ethernet communication between the measurement device (10) and the notification device (50).

7. The measurement system according to any of claims 1 to 6, further comprising a communication device (80) that is configured to perform communication with the measurement device (10) by the second communication method and is configured to perform communication by a third communication method different from the first communication method and the second communication method during communication with the measurement device by the second communication method,
wherein the measurement device (10) is configured to stop communication by the first communication unit during communication with the communication device (80).

8. The measurement system according to any of claims 1 to 7, wherein the terminal notification unit (36) of the terminal device (30) and the notification unit (56) of the notification device (50) are displays.
